# EUROPEAN PATENT APPLICATION

(11) **EP 3 798 215 A1**
(43) Date of publication of application: **31.03.2021**
(21) Application number: 19306203.1
(22) Date of filing: 26.09.2019
(51) Int. Cl.: C07D 405/14, C07D 407/12, C09B 41/00, C09D 11/101

(54) **COVALENT PRINTING BY PHOTO-TRIGGERED CLICK LIGATION**

(71) Applicant: Université de Nantes, 44000 Nantes (FR); Université d'Angers, 49100 Angers (FR); Centre National de la Recherche Scientifique, 75016 Paris (FR)
(72) Inventor: FELPIN, François-Xavier, 44800 Saint-Herblain (FR); DENIS, Marie, 44000 Nantes (FR); GINDRE, Denis, 49000 Angers (FR)
(74) Representative: Novagraaf Technologies

(57) **Abstract**

The present invention relates to the field of covalent printing. More particularly it relates to cyclopropenone derivatives (ink compounds), their use in a covalent printing process and their synthesis process. The invention also relates to a covalent printing process comprising a step of reacting an ink compound according to the invention with a functionalized cellulose sheet comprising -N ₃ functional groups. On other aspects the invention also encompasses covalently printed paper sheets.

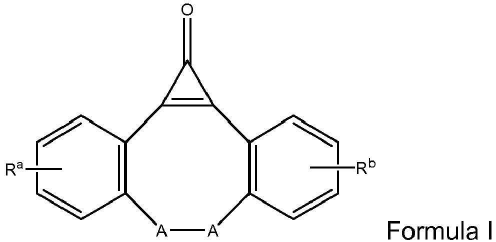

## Description

### Technical field

The present invention relates to the field of covalent printing. More particularly it relates to cyclopropenone derivatives (ink compounds), their use in a covalent printing process and their synthesis process. The invention also relates to a covalent printing process comprising a step of reacting an ink compound according to the invention with a functionalized cellulose sheet comprising -N₃ functional groups. On other aspects the invention also encompasses covalently printed paper sheets.

In the description below, references between **[ ]** refer to the list of references at the end of the examples.

### Technical background

Current printing technologies on cellulose paper mainly relies on the use of inkjet printers on both occupational and domestic settings. Inkjet printing creates patterns by physisorbtion of inks which are typically polymers, but nanoparticles, metal-organic framework and therapeutic drugs can be used as well **[1,2,3,4].** While simplicity of use, modest cost and speed are some of the indisputable assets of inkjet printing, it suffers from the variable stability of physisorbed patterns upon contact with liquids which can be degraded or erased **[5].** This drawback is particularly salient when inkjet printing is used for anti-counterfeiting and tracking applications.

The concept of covalent printing on cellulose paper was recently introduced **[6].** Covalent printing associates traditional features of inkjet printing such as (i) a spatial resolution in the micrometre scale, (ii) a high modularity for use in diverse applications and (iii) a preservation of the whiteness in non-patterned region, with an increased robustness to pattern wash-off due to the covalent linkage between the cellulose paper and the ink. The approach was based on the use of dithiodiglycolic acid as covalently grafted linker which upon light irradiation at 365-400 nm, allows spatio-temporal thiol-X ligation of small molecules as chromophoric or hydrophobic inks **[6,7].** A few number of other methods allowing the covalent functionalization of cellulose paper with a spatial resolution are known, though they do not fill the requirement of covalent printing as they either use organometallic catalysts staining the paper, linkers of low stability or fluorescent linkages in the visible light.

One of the key feature that has to be considered to develop an efficient covalent printing technology, is the time required to print patterns. The first generation of covalent printing using spatio-temporal thiol-X ligation recently developed, requires 150 minutes of printing process (irradiation).

The existing process are therefore too slow to be properly used and industrialised. There is therefore a need to drastically reduce the time required to covalently print patterns on cellulose paper through the establishment of a second generation of covalent printing process.

### Detailed description of the invention

Applicant has surprisingly found out a new way of covalently printing cellulosic paper. The applicant has thus developed a new ink compound that can be easily covalently printed using a photo-triggered click chemistry reaction.

The invention therefore relates to a new ink compound of formula I: 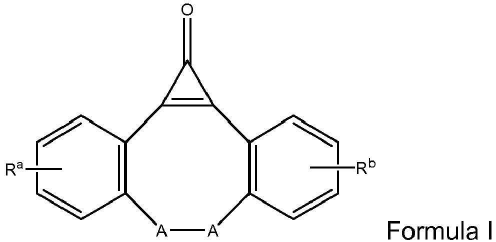
wherein R^{a} and R^{b}, identical or different, represent a C10 to C20 saturated or unsaturated, substituted or non-substituted, aliphatic, heteroaliphatic, cyclic, alicyclic, heteroalicyclic, aryl, heteroaryl, alkylaryl or alkylheteroaryl group, optionally comprising heteroatoms such as N, S or O, comprising a coumarin radical,
each A independently represent CH₂, O, S or NR^{c}, R^{c} being a linear or branched C1 to C8 alkyl chain or a C7 to C9 alkylaryl group.

Advantageously, the ink compound according to the invention may be further chosen from the ink compound of formula I, wherein R^{a} and R^{b}, identical or different, are chosen from the groups of formula II: 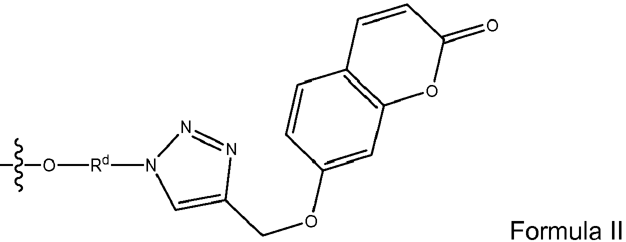 wherein, R^{d} is a linear or branched C1 to C8 alkyl chain. Preferably, R^{d} is -CH₂-CH₂-, -CH₂-CH₂-CH₂- or -CH₂-CH₂-CH₂-CH₂-.

Advantageously, the ink compound according to the invention may be further chosen from the ink compound of formula I, wherein R^{a} and R^{b}, identical or different, are chosen from the groups of formula II': 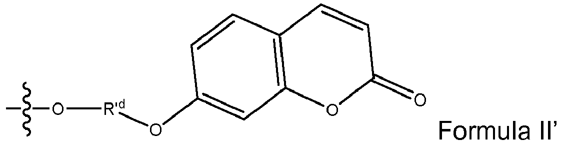 wherein, R'^{d} is a linear or branched C1 to C8 alkyl chain. Preferably, R'^{d} is -CH₂-CH₂-, -CH₂-CH₂-CH₂- or -CH₂-CH₂-CH₂-CH₂-.

Advantageously, the ink compound according to the invention may be further chosen from the ink compounds of formula I, wherein R^{a} and R^{b} identical or different, are chosen from the following groups: 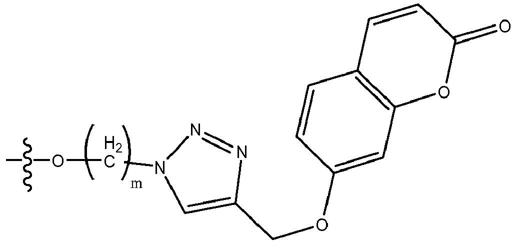 or 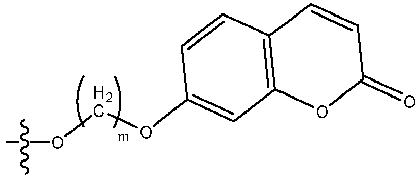 wherein m is an integer from 2 to 6.

Advantageously, the ink compound according to the invention may be further chosen from the ink compounds of formula I, wherein R^{a} and R^{b} identical or different, are chosen from the following groups: 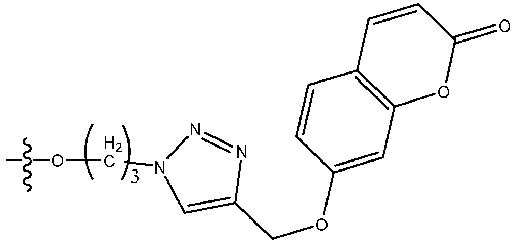 or

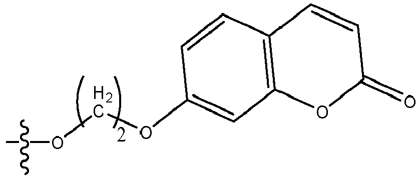

Advantageously, the ink compound according to the invention may be further chosen from the ink compound of formula I, wherein each A independently represents CH₂, O, S or NR^{c}, R^{c} being a linear or branched C1 to C8 alkyl chain or a C7 to C9 alkylaryl group. Preferably, each A is independently chosen from CH₂, O, S or N(CH₂-C₆H₅). More preferably each A represents CH₂

Advantageously, the ink compound according to the invention may be further chosen from the following compounds:

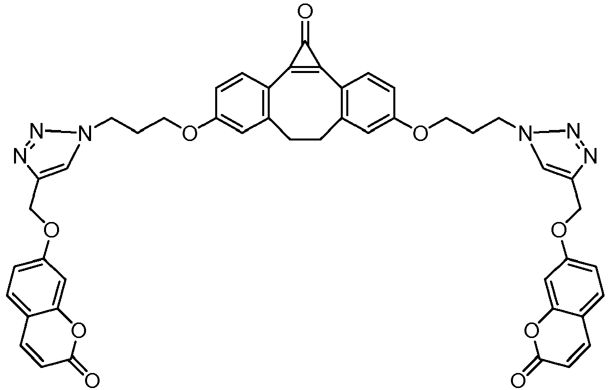

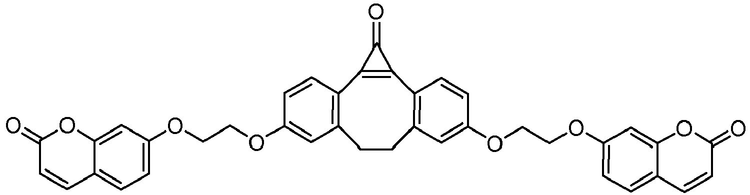

The invention further relates to a covalent printing process comprising a step of reacting an ink compound according to the invention with a functionalized cellulose sheet comprising -N₃ functional groups, and obtaining a covalently printed paper sheet.

It is meant by "paper sheet", a material that may be manufactured in thin sheets from the pulp of wood or other fibrous substances, used for writing, drawing, or printing on, or as wrapping material. It is preferably made from cellulose but may comprise other additives such as starch and carbonates.

Advantageously, the reaction of the ink compound according to the invention with the functionalized cellulose sheet comprising -N₃ functional groups may be a photo-triggered click chemistry reaction, and more particularly a 1,3-dipolar cycloaddition.

It is meant herein by "photo-triggered click reaction", a process where a cyclopropenone undergoes a rapid decarbonylation upon light irradiation to give a reactive cyclooctyne which reacts with an organic azide.

Advantageously, the photo-triggered click chemistry reaction may comprise the steps of:
a) absorbing a solution comprising a solvent and the ink compound of the invention on the surface of the functionalized cellulose sheet,
b) drying the obtained cellulose sheet, and
c) irradiating the dried cellulose sheet with UV light at a wavelength from 250 nm to 370 nm, preferably 365 nm.

Advantageously, the absorption step a) is done by simple deposition of the solution comprising the ink compound using a syringe or any other mean that allows a precise deposition on the cellulose sheet. The absorption may be done for example using a syringe, a nozzle or a printer tip. The duration of the absorption step may be between 1 second and several minutes.

Advantageously, the absorption step a) may only be applied to specific areas of the cellulose sheet that will be covalently printed. This allows to print any letters, shape, motif, design or decoration needed by the user.

Advantageously, the covalent printing process according to the invention may comprise a step of cellulose sheet functionalisation. The cellulose sheet may be functionalised by -N₃ functional groups. The functionalization of the cellulose sheet may be a surface functionalisation. The functionalisation may comprise a tosylation step (it may also be a mesylation or a bromation step, or by any other suitable leaving group). The tosylation step may be done with TsCI in an organic solvent (i.e. pyridine). The duration of the tosylation step may be from 1 hour to 48 hours, preferably 24 hours. The temperature of the tosylation step may be from 30°C to 60°C, preferably 40°C. The sheet may further be rinced with an organic solvent (i.e. DMF). The azoturation step may be done with NaN₃ (usually from 1 to 30 eq, preferably 20 eq) in an organic solvent (i.e. DMF). The duration of the tosylation step may be from 1 hour to 48 hours, preferably 36 hours. The temperature of the tosylation step may be from 40°C to 80°C, preferably 60°C. The paper may further be rinsed with several portions of water and organic solvent (i.e. CH₂Cl₂).

Advantageously, the cellulose sheet -N₃ functionalization rate may be from 10 to 40%, preferably from 20 to 30%.

Advantageously, the -N₃ group may be evenly distributed on the surface of the cellulose sheet. The -N₃ may also be distributed on specific areas, following predefined letters, shape, motif, design or decoration needed by the user. In the latest the cellulose sheet can be considered as pre-printed as the process according to the invention will reveal pre-printed parts.

Advantageously, prior to surface functionalization, the hydrogen bonds may be broken for increasing both the surface area of the paper and the reactivity of hydroxyl groups. The activation step may usually be achieved by soaking cellulose sheet basic aqueous solution. Then the sheet may be rinced with several portions of solvent (for example ethanol) until the pH of the solution is neutral. The duration of the soaking may be from 1 to 48 hours, preferably 24 hours, preferably at room temperature. The basic aqueous solution may be a 10 wt% NaOH aqueous solution.

Advantageously, the ink concentration in the solution of step a) may be from 0.1 mmol/L to 200 mmol/L, preferably from 1 to 10 mmol/L and more preferably 7 mmol/L.

Advantageously, according to the process of the invention, a quantity of ink compound from 0.01 to 0.1 equivalent per -N₃ unit may be used, preferably from 0.04 to 0.1.

Advantageously, the solvent in the solution of step a) may be an organic solvent, preferably chosen in the group comprising chloroform, toluene, acetonitrile, methanol, ethanol, ethyl acetate. Preferably the organic solvent may be chloroform.

Advantageously, the drying step b) is done with any kind of dryer suitable for use with a cellulose sheet. Preferably, the dryer is pulsed air drier such as a thermal cleaner, or the drying step b) is done under vacuum.

Advantageously, the duration of step b) is from 1 second to 1 minute, preferably from 20 to 30 seconds.

Advantageously, the irradiation step c) may be done with a UV lamp emitting at a wavelength from 250 nm to 370 nm, preferably 365 nm. The irradiation step c) may also be done with a LASER.

Advantageously, the duration of the irradiation step c) is less than 20 minutes, preferably less than 10 minutes.

Advantageously, the irradiation step c) may only be applied to specific areas of the cellulose sheet that will be covalently printed. The irradiation step may be controlled by means of a photomask or a light scanning system on the area to be marked. This allows to print any letters, shape, motif, design or decoration needed by the user.

Advantageously, the irradiation step c) may be applied in a way that allows gray-scale covalent printing. For the same irradiation period but using a photomask with different opacity levels: 0%, 25%, 50%, 75%, 75% and 100%, results in a variation in the colour of the marking that is consistent with the photomask used. The process for absorbing, drying and irradiating the paper is strictly the same as described above.

Advantageously, the covalent printing process according to the invention may further comprise a step d) of washing and drying the covalently printed paper sheet. In step d), the washing may be done with an organic solvent. The solvent may be the same as described in the solution in step a).

The invention further relates to a covalently printed paper sheet obtained according to the process of the invention.

The invention also encompasses a covalently printed paper sheet comprising at least one substituent of formula IV: 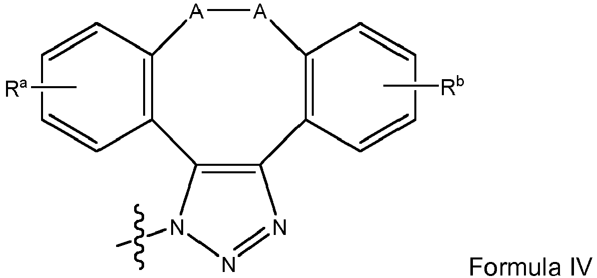 in which A, R^{a} and R^{b} are defined as above.

Advantageously, the position of the groups of formula IV will be determined by the letters, shape, motif, design or decoration that the user wants to print on the cellulose sheet. The letters, shape, motif, design or decoration may be determined in step a), where the solution is absorbed on specific places on the cellulose sheet surface. The user will choose depending on the wanted letters, shape, motif, design or decoration where the solution is absorbed.

Advantageously, the letters, shape, motif, design or decoration may also be determined in step c), where the irradiation is done only on specific places of the cellulose sheet surface. The user will choose depending on the wanted letters, shape, motif, design or decoration where the irradiation will selectively occur.

Advantageously, concentration of the groups of formula IV may also depend on the intensity intended, specifically it may depend on the concentration of solution absorbed in step a), the duration of irradiation in step c) or the gray-scale covalent printing technique that may have been applied.

Advantageously, the covalently printed paper sheet according to the invention may comprise cellulose of formula III: 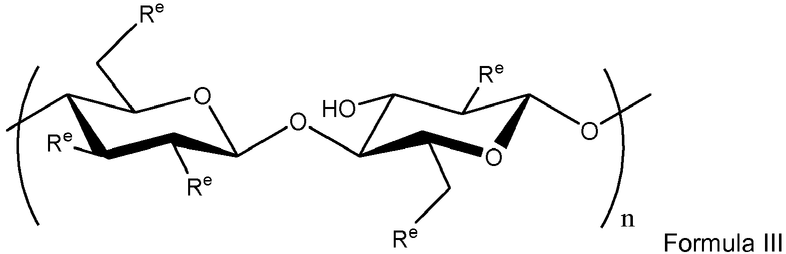 wherein,
n is from 20 to 20000,
each R^{e} independently represents -OH, -N₃ or a substituent of formula IV: 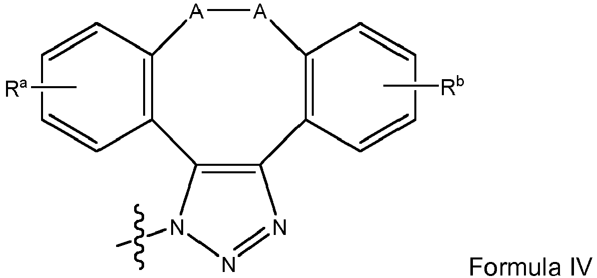
   in which A, R^{a} and R^{b} are defined as above,
   at least one R^{e} is a substituent of formula IV.

The invention further relates to the use of an ink compound according to the invention in a covalent printing process.

The invention is also a synthesis process of an ink compound according to the invention, comprising a step of reacting an intermediate compound of formula la: 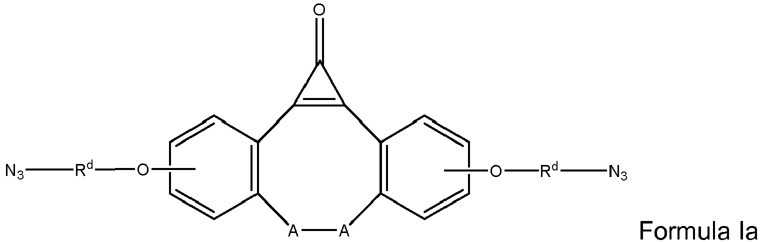 and a compound of formula IIa : 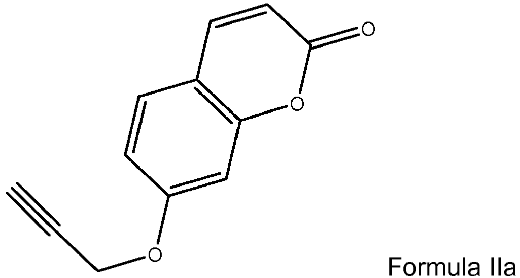 in which A and R^{d} are defined as above.

The invention is also a synthesis process of the intermediate compound of formula la, comprising a step of reacting a compound of Formula Ib: 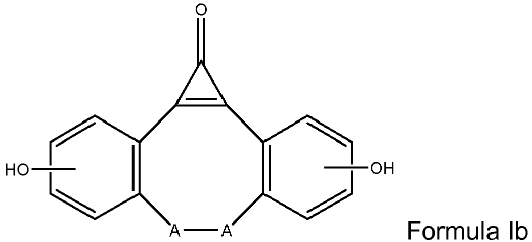 and a compound of formula IIb :

Grp-R^{d}-N₃ Formula IIb

in which,
A and R^{d} are defined as above,
Grp represent a leaving group, preferably OTs, OMs, I, Br.

The invention is also a synthesis process of an ink compound according to the invention, comprising a step of reacting an intermediate compound of Formula Ib: 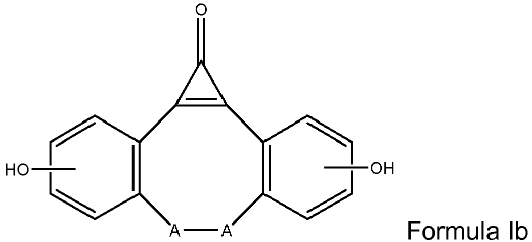 and a compound of formula IIc : 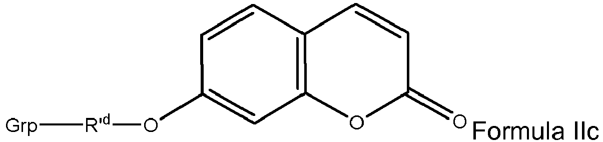 in which,
Grp, A and R'^{d} are defined as above.

### Brief description of the figures

- Figure 1 represents the general strategy of the covalent printing process.

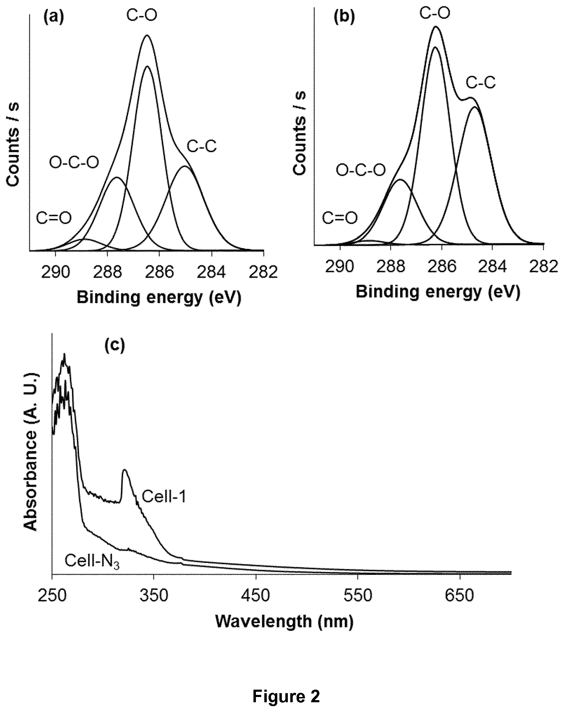

- Figure 2 represents high resolution C1s spectra of (a) Cell-N₃ and (b) Cell-DIBO. UV-vis absorption spectra of Cell-N₃ (violet line), Cell-DIBO (green line).
- Figure 3 represents (a) a curve representing the blue coordinates as a function of the irradiation time and (b) a photograph of pattern at different irradiation times under a UV lamp at 365 nm.
- Figure 4 represents a grayscale printed sheet (using a photomask) according to the invention.

### EXAMPLES

### Example 1: Preparation of ink compounds

### Synthesis of (3-methoxybenzyl)triphenylphosphonium chloride :

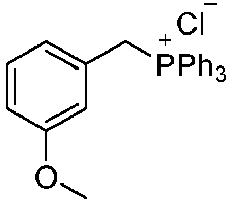

A stirred solution of 3-(chloromethyl)anisole (12 g, 76.62 mmol) in CH₃CN (30 mL) was treated with PPH₃ (30.0 g, 114.38 mmol, 1.49 eq.). The mixture was vigorously stirred, refluxed for 18 hours and then evaporated. The crude compound was purified by precipitation from CHCl₃/Et₂O to give (3- methoxybenzyl) triphenylphosphonium chloride as a white solid (29.50 g, 69.95 mmol, 91.3%).
Mp °C: 274°C^{a}, IR: 2990, 2861, 2781, 1590, 1483, 1464, 1433, 1322,1250, 1156, 1107, 1038, 995, 933, 866, 804, 767, 764 , 715, 688, 619, 534, 502, 447. ¹H NMR (400 MHz, CDCl₃) δ 3.52 (s, 3H), 5.46 (d, *J* = 13.9 Hz), 6.62-6.66 (m, 1H), 6.73-6.76 (m, 2H), 6.98-7.03 (m, 1H), 7.59 - 7.65 (m, 6H), 7.72 - 7.79 (m, 9H), ¹³C NMR (101 MHz, CDCl₃) δ,30.59, 31.21 , 55.51, 115.28, 115.37, 116.35, 116.45, 117.69, 118.81, 123.76, 123.87, 128.83, 128.98, 129.81, 129.86, 130.18, 130.33, 134.58, 134.73, 135.00, 135.06, 159.77, 159.84. MS (TOF ES+): 383.27 [M]+
^{a}lit. **[8]** : 271-272°C

### Synthesis of 1,2 bis (3 methoxyphenyl) ethane

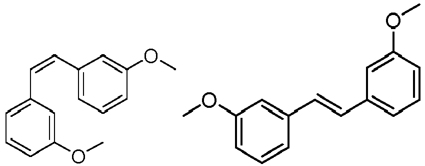

(3-methoxybenzyl)triphenylphosphonium chloride (9.976 g, 28.86 mmol) was dissolved in dry THF (60 mL) under N₂ and cooled to 0 °C. *t*-BuOK (4.28 g, 38.17 mmol, 1.6 eq.) was added in small portions and the mixture was stirred for 90 min at RT. 3-methoxybenzaldehyde (4.61 g, 33.88 mmol, 1.42 eq.) in dry THF (20 mL) was added dropwise at 0 °C. After stirring ON at reflux, the reaction mixture was quenched with 1N HCl (100 mL), and extracted with diethylether (2 x 100 mL). The combined organic phases were dried by anhydrous MgSO₄, and concentrated on a rotary evaporator. The crude product was purified by a silica gel column (95/5 Petroleum ether /Ethyl Acetate) to afford the oil mixed product of *cis* and *trans* 1,2 bis (3 methoxyphenyl)ethene (5.297 g, 22.04 mmol, 92,3%). MS (TOF ES+): 241.12 [M+H]⁺ IR : 3045, 2996, 2961, 2832, 1917, 1587, 1488, 1449, 1339, 1288, 1266, 1238, 1200, 1159, 1080, 1032, 973, 939, 853, 777, 729, 689, 621, 560, 537, 484, 455,
(Z): ¹H NMR (300 MHz, CDCl₃) δ: 3.67 (s, 6H), 6.58 (s, 2H), 6.73-6.77 (m, 2H), 6.79 - 6.81 (m, 2H), 6.84 - 6.87 (m, 2H), 7.15 (t, *J* = 7.99 Hz, 2H). ¹³C NMR (75 MHz, CDCl₃) δ: 55.19, 113.42, 113.95, 121.59, 129.33, 130.46, 138.63, 159.58.
(E): ¹H NMR (300 MHz, CDCl₃) δ 3.86 (s, 6H), 6.80-6.85 (m, 2H), 7.04-7.05 (m, 2H), 7.08 (s, 2H), 7.10-7.13 (m, 2H), 7.28 (t, *J* = 7.8 Hz, 2H), ¹³C NMR (75 MHz, CDCl₃) δ 55.41, 111.90, 113.56, 119.45, 129.08, 129.80, 138.82, 160.0.

### Synthesis of 1,2 bis (3-methoxyphenyl) ethane

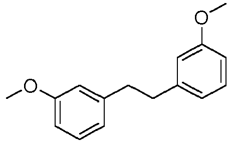

A suspension of 1,2 bis (3-methoxyphenyl)ethene (5.5 g, 22.89 mmol) and 10% Pd/C (1.1g, 20% w/w) in methanol (50 mL) was stirred at room temperature ON under H₂ atmosphere (1 atm). The reaction mixture was then filtered over celite and the filtrate was concentrated under vacuum. The **residue** was purified by flash chromatography on silica gel using a mixture of petroleum ether and ethyl acetate (6/1) affording pure alkane as a colorless oil (5.25 g, 21.66 mmol, 94.7%). IR: 3003, 2972, 2923, 2842, 1930, 1590, 1467, 1346, 1317, 1272, 1247, 1191, 1161, 1075, 1033, 965, 920, 884, 859, 785, 694, 617, 552, 460. MS (TOF ES+): 241.122 [M+H]⁺. ¹H NMR (400 MHz, CDCl₃) δ 2.90 (s, 4H), 3.79 (s, 6H, OCH₃), 6.74-6.81 (m, 6H, Ar), 7.18-7.22 (t, *J* = 7.6 Hz, 2H, Ar,). ¹³C NMR (MHz, CDCl₃) δ 37.94, 55.28, 111.48, 114.35, 121.02, 129.43, 143.59, 159.88.

### Synthesis of 4,9-dimethoxy-6,7-dihydro-1H-dibenzo[a,e]cyclopropa[c]-[8]annulen-1-one

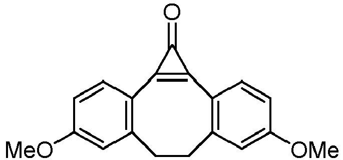

Tetrachlorocyclopropenone (2.90 g, 16.342 mmol, 0.99 eq.) and AlCl₃ (3.3 g, 24.76 mmol, 1.5 eq.) were dissolved in dry CH₂Cl₂ (80 mL) and cooled to -78 °C. A solution of 1,2 bis (3-methoxyphenyl)ethane (4.0 g, 16.50 mmol, 1 eq.) in dry CH₂Cl₂ (20 mL) was added dropwise. The resulting mixture was stirred for 2 hours at -78 °C, for 1 h at 24 °C, and quenched with ultra-pure water (100 mL). It was filtered through a pad of Celite, which was rinsed with several portions of CH₂Cl₂. The organic layers were washed with brine (100 mL), dried over anhydrous MgSO4 and evaporated under reduced pressure to give a crude product which was purified by silica gel column with CH₂Cl₂ and methanol (50/1, v/v) as the eluent to afford 1,2 bis (3-methoxyphenyl)ethane (3.3 g, 11.29 mmol, 68%) as a white solid. Mp °C : 182°C, MS (TOF ES+): 293.117[M+H]⁺ , IR : 3914, 3884, 3781, 3699, 3662, 3635, 3407, 2391, 2076, 1843, 1725, 1664, 1601, 1556, 1424, 1339, 1314, 1269, 1135, 1048, 1018, 944, 867, 807, 729, 613, 561, 518, 465, 445. ¹H NMR (400 MHz, CDCl₃) δ 2.64 (d, J=10.2Hz, 2H), 3.35 (d, J= 10.2 Hz, 2H), 3.89 (s, 6H), 6.90-6.92 (m, 4H), 7.94-7.96 (m, 2H).¹³C NMR (101 MHz, CDCl₃) δ 37.42, 55.67, 111.99, 115.96, 116.68, 135.97, 142.53, 147.95, 153.90, 162.61.

### Synthesis of 4,9-dihydroxy-6,7-dihydro-1H-dibenzo[a,e]cyclopropa[c]-[8]annulen-1-one

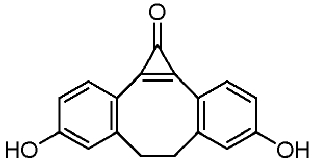

4,9-dimethoxy-6,7-dihydro-1H-dibenzo[a,e]cyclopropa[c]-[8]annulen-1-one (4.5 g, 15.405 mmol) was dissolved in dry CH₂Cl₂ (180 mL) and cooled to - 78 °C. After BBr₃ (23.156 g, 92.431 mmol, 6 eq.) was added dropwise and the resulting mixture stirred for 30 min at -78 °C and then for 4h at 24°C. After cooled to 0°C, the reaction was quenched with MeOH (150 mL) and then concentrated to a brown paste. The residue was suspended in absolute ethanol (15 mL), stirred for 15 min and then filtered to afford the title compound as a tan solid (2.5g, 9.46 mmol, 61%). MS (TOF ES+): 287.068 [M+Na]⁺, IR : 3222, 3068, 3028, 2975, 2806, 2347, 2293, 2121, 1929, 1833, 1663, 1603, 1568, 1524, 1484, 1446, 1363, 1342, 1309, 1286, 1225, 1163, 1133, 1080, 972, 867, 830, 815, 754, 737, 693, 629, 611, 544, 471, 443. ¹H NMR (400 MHz, DMSO) δ 2.43 (d, J= 9.8 Hz, 2H), 3.35-3.33 (m, 2H), 6.81- 6.84 (dd, J=2.6Hz, J=8.2 Hz, 4H), 6.88 (d, J= 2.6 Hz, 2H); 7.68 (d, *J* = 8.4 Hz, 2H), 10.36 (s, 2H). ¹³C NMR (101 MHz, DMSO) δ 36.37, 113.88, 114.61, 116.88, 134.98, 141.13, 148.03, 151.9, 160.84

### Synthesis of 3-Azidopropanol :

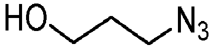

Sodium azide (5.50 g, 2 eq.) was dissolved into 65 mL of water. 3 chloropropan-1-ol (4 g, 3.54 mL, 1 eq.) was added slowly and the medium was stirred and refluxed for 24h then allowed to RT. The medium was extracted with CH₂Cl₂ (3 × 80 mL). The organic phases were dried over anhydrous Na₂SO₄, filtered and concentrated. 3-azidopronaol was obtained as a colorless oil (3.58 g, 35.42 mmol, 83.7%). IR: 3336, 2945, 2881, 2090, 1454, 1343, 1258, 1054, 955, 900, 637, 556, 513, ¹H NMR (400 MHz, CDCl₃) δ 1.49 (s, 1H), 1.84 (quintet, J = 6.75 Hz, 2H), 3.45 (t, *J* = 6.75 Hz, 2H), 3.76 (m, 2H). ¹³C NMR (CDCl₃) δ 31.70, 48.73, 60.17.

### Synthesis of 3-Azidopropyl 4-methylbenzenesulfonate :

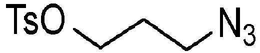

3-azidopropan-1-ol (3.58 g, 35.42 mmol) was dissolved in CH₂Cl₂ (160 mL). Et₃N (5.37 g, 53.14 mmol, 1.5 eq.) was added and the mixture was cooled to 0°C under argon atmosphere. Then, TsCl (8.10 g, 42.51 mmol, 1.2 eq.) was added and the medium was stirred at 0 °C for one hour and allowed to RT for 18 hours. The organic phase was washed with 160 mL of NaHCO₃ saturated solution, 160 mL of water and 160 mL of NaCl saturated solution. The organic phase was dried over Na₂SO₄, filtered and concentrated. The crude was purified over silica gel (EP/AcOEt 95/5 to eliminate the TsCl then 70/30) to obtain: 3-Azidopropyl 4-methylbenzenesulfonate as a colorless oil (6.95 g, 27.26 mmol, 76.95%). MS (TOF ES+): 278.057 [M+Na]⁺, IR : 2929, 2095, 1597, 1494, 1455, 1356, 1292, 1210, 1188, 1174, 1097, 1019, 978, 942, 814, 751, 705, 688, 664, 576, 555. ¹H NMR (400 MHz, CDCl₃) δ 1.90 (quintet, *J* = 6.34 Hz, 2H), 2.46 (s, 3H), 3.37 (t, *J* = 6.47 Hz, 2H), 4.12 (t, *J* = 6.03 Hz, 2H), 7.36 (d, *J* = 8.0 Hz, 2H), 7.80 (d, *J* = 8.3 Hz, 2H). ¹³C NMR (101 MHz, CDCl₃) δ 21.84, 28.93, 47.78, 67.19, 128.22, 130.28, 133.26, 145.16.

### Synthesis of 3-Azidopropyl DIBO

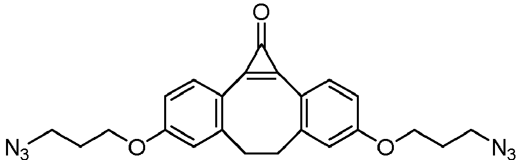

4,9-dimethoxy-6,7-dihydro-1H-dibenzo[a,e]cyclopropa[c]-[8]annulen-1-one (1.5 g, 5.676 mmol) was added in dry DMF (90 mL). Next, anhydrous K₂CO₃ (10.198 g, 73.785 mmol, 13 eq.) and 3-Azidopropyl 4-methylbenzenesulfonate (5.795 g, 22.703 mmol, 4 eq.) were added and the solution was stirred and heated to 45 °C for 4 hours. The crude medium was extracted with Et₂O (4 × 50mL) and washed with a saturated aqueous solution of NaCl (2 × 50mL). The organic phases were dried over anhydrous MgSO₄, filtered and evaporated. Column chromatography (100% AcOEt) afforded the compound as a yellowish solid (1.96 g, 4.55 mmol, 80%). Mp °C: 81-82 °C, MS (TOF ES+): 431.183 [M+H]⁺, IR = 3059, 3029, 2944, 2880, 2199, 2192, 2097, 1840, 1763, 1599, 1563, 1501, 1489, 1471, 1420, 1392, 1372, 1346, 1334, 1307, 1291, 1271, 1244, 1188, 1051, 987, 975, 937, 918, 861, 817. ¹H NMR (400 MHz, CDCl₃) δ 2.09 (quintet, J= 6.24 Hz, 4H), 2.64 (d, *J* = 10.6 Hz, 2H), 3.35 (d, *J* = 10.6 Hz, 2H), 3.54 (t, *J* = 6.3 Hz, H), 4.14 (t, *J* = 5.9 Hz, 4H), 6.90 (m, 4H), 7.96 (m, 2H). ¹³C NMR (101 MHz, CDCl₃) δ 28.98, 37.46, 48.37, 65.07, 112.65, 116.49, 117.02, 136.15, 142.73, 148.17, 154.04, 161.82.

### Synthesis of 7-propargyloxycoumarine

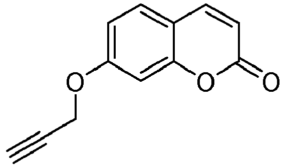

To a solution of umbelliferone (3.0 g, 18.5 mmol) in dry DMF (24 mL) was added anhydrous potassium carbonate (5.114 g, 37.00 mmol, 2 eq.) under an inert atmosphere. Then, propargyl bromide (2.641 g, 22.20 mmol 1,2 eq.) was added to the solution and stirred at RT for 4 hours. After the completion of the reaction, the solution was quenched with H₂O (180 mL) and extracted with AcOEt (150 mL). The organic layer was washed with brine (4 × 90mL), dried over anhydrous Na₂SO₄, filtered and concentrated under reduced pressure. Mp °C: 118-119°C^{a}, MS (TOF ES+): 205.055 [M+H]⁺, IR : 3273, 1698, 1609, 1502, 1469, 1427, 1400, 1342, 1269, 1230, 1187, 1155, 1125, 1010, 976, 923, 892, 826, 754, 715, 683, 635, 617, 512, 470, 451, ¹H NMR (300 MHz, DMSO) δ 3.66 (t, *J* = 2.4 Hz, 1H), 4.94 (d, *J* = 2.4 Hz, 2H), 6.32 (d, *J* = 9.5 Hz, 1H), 6.99 (dd, *J* = 8.6, 2.5 Hz, 1H), 7.05 (d, *J* = 2.4 Hz, 1H), 7.65 (d, *J* = 8.6 Hz, 1H), 8.00 (d, *J* = 9.6 Hz, 1H). ¹³C NMR (75 MHz, DMSO) δ 56.06, 78.47, 78.89, 101.74, 112.84, 112.93, 129.48, 144.19, 155.09, 160.13, 160.16.
^{a}litt **[9]** : 118-120°C

### Synthesis of the ink compound 1:

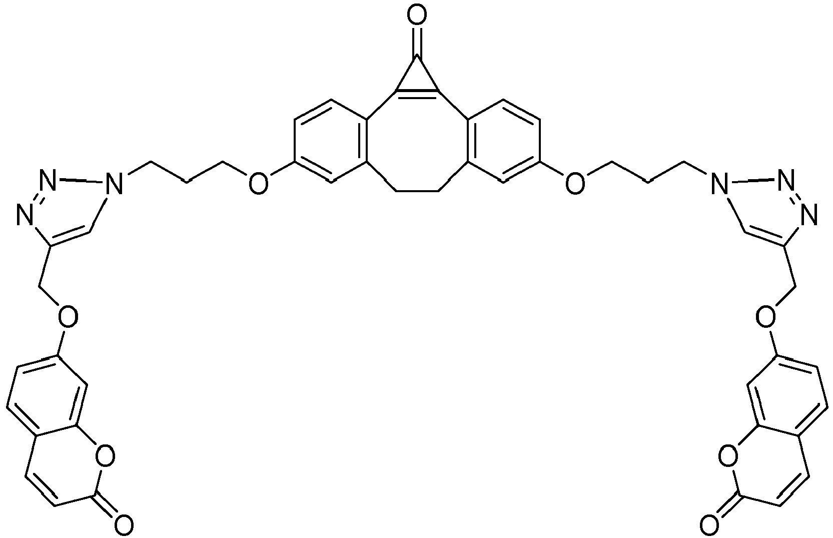

To a solution of propargylcoumarine (0.409 g, 2.044 mmol, 8.8 eq.) in *t-*BuOH/H2O (1:1) (10 mL), CuSO₄.5H₂O (9 mg, 0.035 mmol, 0.2 eq.) and sodium ascorbate (14 mg, 0.07 mmol, 0.3 eq.) were added. The mixture was stirred at room temperature for 15 min and the azide (0.1 g, 0.232 mmol, 1 eq.) was added. The resulting mixture was stirred at 50 °C for 24 hours. After 24 h, the reaction mixture was extracted with CH₂Cl₂ (2 × 30mL). The combined organic phases were washed with water (10 mL), dried over anhydrous sodium sulphate and evaporated in vacuum. The obtained solid was dissolved in MeOH, filtered and rinced with several portions of acetone. The obtained solid was dried under reduce pressure to obtain a pale yellow product (0.130 g, 0.16 mmol, 67%). Mp °C: 228°C, MS (TOF ES+): 831.277 [M+Na]⁺, IR : 3134, 3089, 2937, 2872, 2103, 1985, 1837, 1710, 1604, 1559, 1504, 1457, 1428, 1401, 1348, 1316, 1293, 1281, 1248, 1228, 1204, 1129, 1100, 1047, 1008, 981, 890, 828, 754, 704, 686, 659, 634, 616, 579, 543, 507, 459, 426, ¹H NMR (500 MHz, Sonde CRYO DUAL, DMSO d6) : δ 2.31-2.36 (m, 4H), 2.45 (br, 2H), 3.40 (m, 2H), 4.11 (br, 4H), 4.58 (t, *J* = 6.6 Hz, 4H), 5.27 (s, 4H), 6.29 (d, *J* = 9.5 Hz 2H), 6.99 (dq, *J* = 2.8 Hz, 8.7 Hz, 19.3 Hz, 4H), 7.07 (d, *J* = 2.3 Hz, 2H), 7.15 (d, *J* = 2.3Hz, 2H), 7.63 (d, *J*=8.4 Hz, 2H), 7.77 (d, *J* = 8.4 Hz, 2H), 7.98 (d, *J* = 9.6 Hz, 2H), 8.33 (s,2H). ¹³C NMR (500 MHz, DMSO d6) δ : 29.26, 36.25, 46.56, 61.69, 64.99, 101.52, 112.55, 112.64, 112.80, 112.87, 116.06, 116.12, 124.95, 129.46, 134.88, 141.95, 142.04, 144.20, 147.89, 151.96, 155.27, 160.17, 160.82, 161.09.

### Synthesis of 7-(2-Hydroxyethoxy)-2H-chromen-2-one

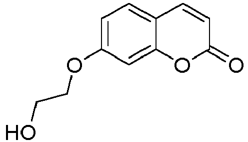

A solution of 7-hydroxycoumarin (1.0 g, 6.1 mmol), dry potassium carbonate (3.96 g, 28.6 mmol), and acetone (24 ml) was heated at 80°C. Then bromoethanol (1.4 ml, 30 mmol) was added in three occasions (every 10 hours). After 40 hours of reaction, water (20mL) was added to the cooled reaction medium. The mixture was extracted twice with CH₂Cl₂. The combined organic phases were washed with brine and concentrated. The crude medium was purified over silica gel with a mixture of AcOEt and cyclohexane (90/10) affording the desired compound as a white solid with a 78% yield. MS (TOF ES+): 229.04 [M+Na]. ¹H NMR (300 MHz, CDCl₃): 1.98 (t, 1H), 4,02 (m, 2H), 4.15 (t, 2H), , 6.26 (d, 1H, J= 9.5 Hz), 6.83-6.89 (m, 2H,), 7.38 (d, 1H), 7.64 (d, 1H). ¹³C NMR (300 MHz, CDCl₃) δ : 61.13, 69.97, 101.82 ; 112.91, 113.63, 129.01 , 143.45, 155.96, 161.20, 162.06

### Synthesis of 7-(2-tosyl-oxyethoxy)-2H-chromen-2-one

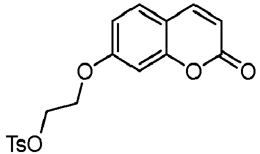

To a solution of 7-(2-Hydroxyethoxy)-2H-chromen-2-one (0.5 g, 2.27 mmol) in anhydrous CH₂Cl₂ (10 mL) was added anhydrous pyridine (10 mL) and p-toluenesulfonyl chloride (1.29 g, 6.8 mmol) and the mixture was stirred for 16 h under an inert atmosphere at room temperature. The reaction was quenched by saturated aqueous sodium bicarbonate (20 mL) and extracted with CH₂Cl₂ (2 × 30 mL). The combined organic layers were washed with saturated sodium chloride solution (2 × 15 mL) and dried over MgSO₄. MS (TOF ES+): 383.1 [M+Na]⁺, 361.1 [M+H]. ¹H NMR (400 MHz, CDCl₃): 2.45 (s, 3H), 4.20 (m, 2H), 4.40 (m, 2H), 6.26 (d, 1H, *J* = 9.5 Hz), 6.67 (d, *J* = 2.29 Hz, 1H), 6.75 (dd, J = 2.29 Hz, *J* = 8.61 Hz, 1H), 7.35 (m, 3H),7.61 (d, *J* = 9.52 Hz, 1H), 7.81 (d, *J* = 8.06 Hz, 2H). ¹³C NMR (101 MHz, CDCl₃) δ: 22.16, 66.05, 67.76, 101.89, 112.77, 113.31, 113.81, 128.18, 129.08, 130.06, 133.00, 143.32, 145.33, 155.81, 161.04, 161.24.

### Synthesis of ink compound 2

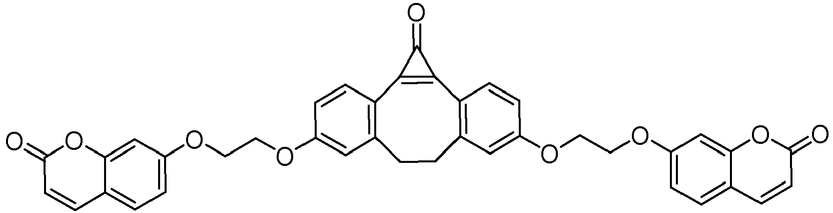

To a solution of photo-DIBO-OH (0.03 g, 0.114 mmol) in dry DMF (2 mL) was added 7-(2-tosyl-oxyethoxy)-2H-chromen-2-one (0.245 g, 0.681 mmol) . Next, K₂CO₃ (0.204 g, 1.476 mmol) was added and the solution was stirred and heated to 45 °C for 48 hours. The reaction concentrated under vacuum and purified over silica gel with AcOEt then CH₂Cl₂ 90 / MeOH 10. The pure product was obtained by precipitation in MeOH with 11% yield (8 mg) as a white solid MS (TOF ASAP+): 641.18 [M+H]⁺. ¹H NMR (700 MHz, CDCl₃): 2.65 (d, 2H), 3.36 (m, 2H), 4.44 (br, 8H), 6.28 (d, 2H, J= 9.32 Hz), 6.89-6.98 (m, 8H), 7.4 (d, J=9.32 Hz, 2H), 7.65 (d, *J* = 9.32 Hz, 2H), 7.98 (d, *J* = 8.94 Hz, 2H). ¹³C NMR (176.1 MHz, CDCl₃) δ: 29.85, 37.27, 66.51, 67.06, 101.168, 112.45, 113.13, 113.67, 116.67, 117.15, 129.08, 136.06, 142.78, 143.53, 148.02, 153.88, 156.07, 161.25, 161.38, 161.79.

### Example 2: Preparation of azide paper

Prior to surface functionalization, breaking hydrogen bonds is an essential step for increasing both the surface area of the paper and the reactivity of hydroxyl groups. The activation step was achieved by soaking cellulose paper for 24 hours at room temperature in 10 wt% NaOH aqueous solution. Then the paper is rinced with several portions of ethanol until the pH of the solution is neutral. The cellulose is soaked in pyridine and the tosylation step is accomplished with TsCl (3 eq.) in pyridine as solvent for 24 hours at 40°C.

The paper is rinced with DMF and the azoturation step occurs with NaN₃ (20 eq.) in DMF for 36 hours at 60°C. The paper is rinced with several portions of water and CH₂Cl₂. The successful coating is secured by elemental analysis. A good degree of substitution was determined (ca 23-24 %) and a paper suitable for covalent printing application was observed. Fourier Transform Infrared spectroscopy (FTIR) also confirmed the grafting : the band in the region of 2105 cm⁻¹ accounts for the -N₃ moiety. The resulting paper **Cell-N₃** is obtained.

### Example 3: Covalent printing

Ink **1**, in solution in CHCl₃ (6 g/L), was adsorbed on the surface of Cell-N₃, dried and subsequently irradiated with UV light at 365 nm for 8 minutes. The resulting paper is washed with CHCl₃ and dried to obtained printed paper **Cell-1.**

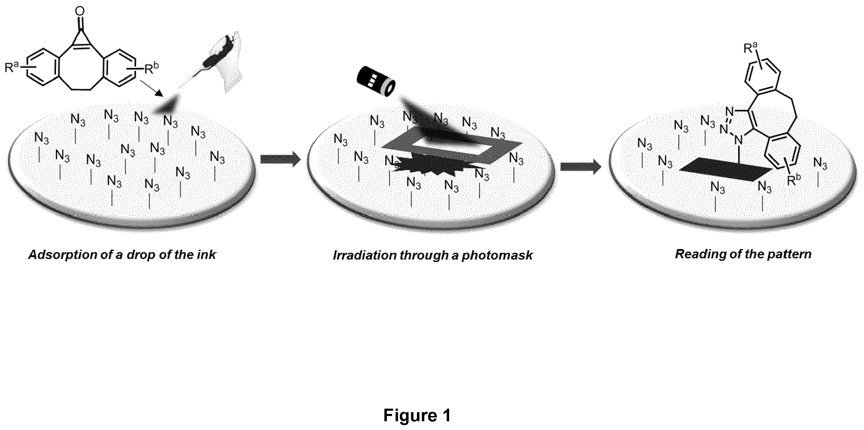

The covalent attachment of **ink 1** to **Cell-N₃** was followed by X-ray photoelectron spectrometry (XPS) and UV-Vis spectrometry. The high resolution C1s spectrum of **Cell-N₃** and **Cell-1** are depicted in Figure 1a-b. Upon light irradiation, the component corresponding to C-C bonds at 284.7 eV significantly increased, attesting to the successful covalent attachment of **ink 1.** The UV-visible spectrum of **Cell-1** also clearly evidenced the characteristic band of the coumarin units at 321 nm. When the light irradiation was omitted, the absorption band at 321 nm could not be detected at the surface of cellulose paper after a single washing with CHCl₃. This experimental evidence is an irrefutable proof for the light-promoted covalent attachment of ink **1** to **Cell-N₃.**

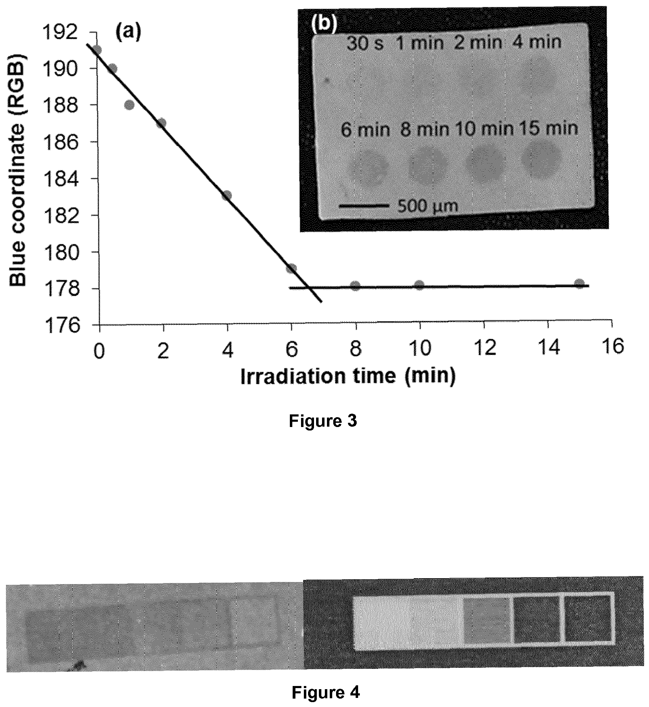

A hidden patterning was realized by irradiation of **ink 1** adsorbed on Cell-N₃ through a shadow mask with a LED (3 mW/cm²) emitting at 365 nm. A strongly fluorescent pattern was revealed under a simple UV-lamp at 365 nm. Importantly, unexposed **ink 1** was unaffected and could be recovered through a single washing of the cellulose paper with CHCl₃ for a further reuse. The time required for the covalent printing and the amount of **ink 1** adsorbed on **Cell-N₃** were carefully optimized as these factors determine for a large part further prospective developments on a more advanced technology readiness level. For instance, we were able to adsorb **ink 1** on **Cell-N₃** at a concentration as low as *ca*. 0.70 µmol/cm². As the concentration of azide functions on **Cell-N₃** was calculated by elemental analysis to be ca. 17 µmol/cm², only 0.04 equiv. of **ink 1** per -N₃ units was needed to create a fluorescent pattern. Glycosidic elements from cellulose mostly dominate the spectral signature in FTIR spectra, and the intensity of the band at 2104 cm⁻¹, corresponding to the vibration band *vₐₛ*(N₃), is not significantly modified by the grafting since at least 96% of azide functions in patterned area are not affected. The irradiation time was optimized in the range of 30 seconds to 15 minutes by analysis of the Red-Blue-Green (RGB) coordinates of circular patterns of *ca*. 12.5 mm². The RGB color model is a universal Cartesian coordinate system for electronic device. The RGB color coordinates were extracted from a picture of the patterned paper with the open-source GIMP raster graphics editor on a square region of the patterned areas of approximately 6 mm². The plot of the blue coordinates versus irradiation time showed a linear decrease from 0 to 6 min and reached a plateau for irradiation times > 8 minutes (Figure 3).

### Example 4: Gray-scale covalent printing

The method allow gray-scale covalent printing, using a photomask, as illustrated in figure 4: left: photo of the gray-scale printing upon illumination with a UV lamp at 365 nm. Right, photo of the photomask.

### List of references

**[1]** B. J. de Gans, P. C. Duineveld and U. S. Schubert, Adv. Mater., 2004, 16, 203-213.
**[2]** T. Ohlund, J. Ortegren, S. Forsberg and H.-E. Nilsson, Appl. Surf. Sci., 2012, 259, 731-739.
[3] L. L. da Luz, R. Milani, J. F. Felix, I. R. B. Ribeiro, M. Talhavini, B. A. D. Neto, J. Chojnacki, M. O. Rodrigues and S. A. Junior, ACS Appl. Mater. Interfaces, 2015, 7, 27115-27123.
[4] (a) N. Sandler, A. Määttänen, P. Ihalainen, L. Kronberg, A. Meierjohann, T. Viitala and J. Peltonen, J. Pharm. Sci., 2011, 100, 3386-3395; (b) R. D. Boehm, P. R. Miller, J. Daniels, S. Stafslien and R. J. Narayan, Mater. Today, 2014, 17, 247-252.
[5] J. Finnell, Library & Archival Security, 2011, 24, 19-24.
[6] J. Rull-Barrull, M. d'Halluin, E. Le Grognec and F.-X. Felpin, J. Mat. Chem. C, 2017, 5, 5154-5162.
[7] G. Bretel, J. Rull-Barrull, M. C. Nongbe, J.-P. Terrier, E. Le Grognec and F.-X. Felpin, ACS Omega, 2018, 3, 9155-9159.
[8] Gulakova, E. N.; Sitin, A. G.; Kuz'mina, L. G.; Fedorova, O. A. Synthesis and Structure of Styryl-Substituted Azines. Russ. J. Org. Chem. 2011, 47 (2), 245-252. https://doi.org/10.1134/S107042801102014X.
**[9]** Kosiova, I.; Kovackova, S.; Kois, P. Synthesis of Coumarin-Nucleoside Conjugates via Huisgen 1,3-Dipolar Cycloaddition. Tetrahedron 2007, 63 (2), 312-320. https://doi.org/10.1016/j.tet.2006.10.075.

## Claims

1. An ink compound of formula I: 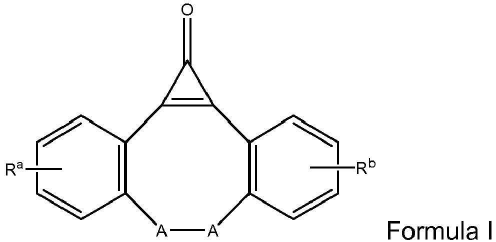
wherein R^{a} and R^{b}, identical or different, represent a C10 to C20 saturated or unsaturated, substituted or non-substituted, aliphatic, heteroaliphatic, cyclic, alicyclic, heteroalicyclic, aryl, heteroaryl, alkylaryl or alkylheteroaryl group, optionally comprising heteroatoms such as N, S or O, comprising a coumarin radical,
each A independently represent CH₂, O, S or NR^{c}, R^{c} being a linear or branched C1 to C8 alkyl chain or a C7 to C9 alkylaryl group.

2. Ink compound according to claim 1, wherein R^{a} and R^{b}, identical or different, are chosen from the groups of formula II: 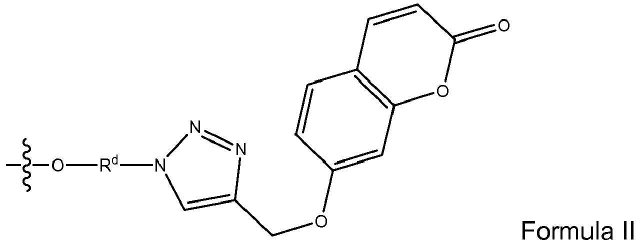 wherein,
R^{d} is a linear or branched C1 to C8 alkyl chain.

3. Ink compound according to claim 1, wherein R^{a} and R^{b}, identical or different, are chosen from the groups of formula II': 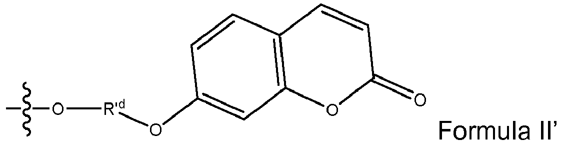 wherein,
R'^{d} is a linear or branched C1 to C8 alkyl chain.

4. Covalent printing process comprising a step of reacting an ink compound according of any of claims 1 to 3 with a functionalized cellulose sheet comprising -N₃ functional groups, and obtaining a covalently printed paper sheet.

5. Process according to claim 4, wherein the reaction is a photo-triggered click reaction.

6. Process according to claim 5, wherein the photo-triggered click reaction comprises the steps of:
a) absorbing a solution comprising a solvent and the ink compound of formula I on the surface of the functionalized cellulose sheet,
b) drying the obtained cellulose sheet, and
c) irradiating the dried cellulose sheet with UV light at a wavelength from 250 nm to 370 nm, preferably 365 nm.

7. Process according to the preceding claim, wherein the duration of the irradiation step c) is less than 20 minutes, preferably less than 10 minutes.

8. Process according to claims 6 or 7, wherein the solvent is an organic solvent, preferably chosen in the group comprising chloroform, toluene, acetonitrile, methanol, ethanol, ethyl acetate, preferably chloroform.

9. Process according to any of claims 6 to 8, wherein the ink concentration in the solution of step a) is from 0.1 mmol/L to 200 mmol/L, preferably from 1 to 10 mmol/L and more preferably 7 mmol/L.

10. Process according to any of claims 6 to 9, wherein the cellulose sheet -N₃ functionalization rate is from 10 to 40%, preferably from 20 to 30%.

11. Process according to any of claims 6 to 10, further comprising a step of washing and drying the covalently printed paper sheet.

12. Covalently printed paper sheet obtained according to the process of any of claims 4 to 11.

13. Covalently printed paper sheet comprising at least one substituent of formula IV: 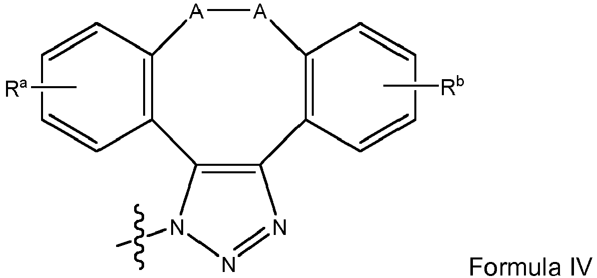 in which A, R^{a} and R^{b} are defined as above.

14. Use of an ink compound according to any of claims 1 to 3 in a covalent printing process.

15. Synthesis process of an ink compound according to claim 2, comprising a step of reacting an intermediate compound of formula la: 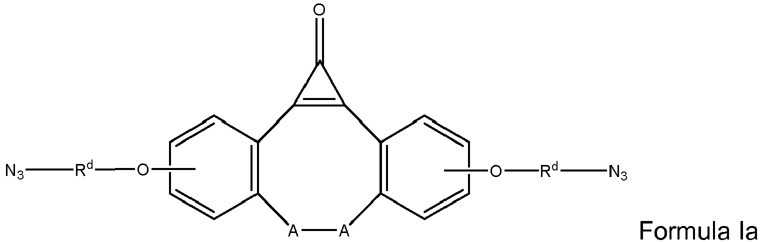 and a compound of formula IIa : 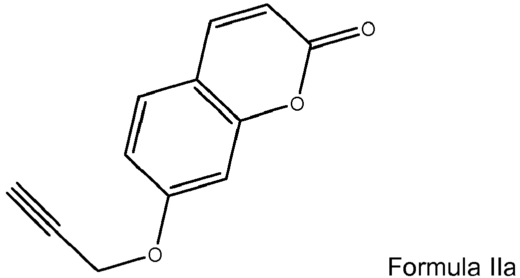 in which A and R^{d} are defined as above.

16. Synthesis process of the intermediate compound of formula Ia according preceding claim, comprising a step of reacting a compound of Formula Ib: 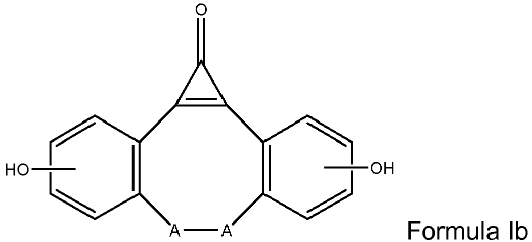 and a compound of formula IIb :
Grp-R^{d}-N₃ Formula IIb
in which,
A and R^{d} are defined as above,
Grp is a leaving group, preferably OTs, OMs, I, Br.

17. Synthesis process of an ink compound according to claim 3, comprising a step of reacting an intermediate compound of Formula Ib: 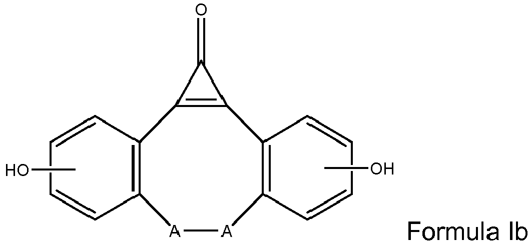 and a compound of formula IIc : 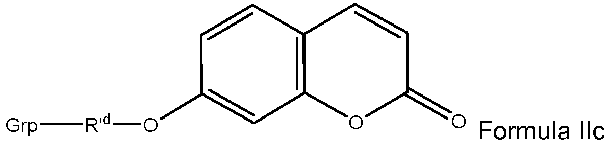 in which Grp, A and R'^{d} are defined as above.
